# EUROPEAN PATENT SPECIFICATION

(11) **EP 2 234 950 B1**
(45) Date of publication and mention of the grant of the patent: **03.09.2014**
(21) Application number: 08863532.1
(22) Date of filing: 22.12.2008
(51) Int. Cl.: C07C 37/72, C07C 45/80, C07C 39/04, C07C 49/08

(54) **REMOVAL OF A SALT FROM A SALT-CONTAINING ORGANIC MIXTURE**
ENTFERNUNG VON SALZ AUS EINER SALZHALTIGEN ORGANISCHEN MISCHUNG
ELIMINATION D'UN SEL SE TROUVANT DANS UN MELANGE ORGANIQUE CONTENANT UN SEL

(30) Priority: 20.12.2007 EP 07150218
(43) Date of publication of application: 06.10.2010
(73) Proprietor: Borealis Technology Oy, 06101 Porvoo (FI)
(72) Inventor: PENTTI, Ismo, 1210 Wien (AT); SYRI, Jari, 06400 Porvoo (FI); SAUKONOJA, Jouni, 06400 Porvoo (FI)
(74) Representative: Golding, Louise Ann
(86) International application number: PCT/EP2008/011010
(87) International publication number: WO 2009/080343

(56) References cited:
- GB-A- 743 004

## Description

The present invention concerns a process for the removal of a salt from a salt-containing organic mixture. Preferably, this process is used to remove a salt from a solution obtained in a phenol production process, for example a solution produced following acid-catalysed cleavage of cumene hydroperoxide (CHP).

Phenol is commonly manufactured through a curnene procedure, wherein cumene is oxidized to cumene hydroperoxide (CHP) and the resulting oxidation product mixture is concentrated and subjected to an acid catalyzed cleavage reaction. Subsequently, the cleavage product mixture is conducted to a distillation section, wherein the main products of the cleavage reaction, i.e. phenol and acetone, are first separated and then purified through a series of further distillation steps or other purification steps.

The cleavage product mixture from the acid-catalyzed cleavage of CHP is acidic due to the presence of the acid catalyst and organic acids formed in side reactions during the oxidation of cumene and/or under the conditions of the subsequent cleavage reaction. The organic acids may include, for example, formic acid and benzoic acid. Before the distillation of the main products of the cleavage reaction (i.e. phenol and acetone), these acids have to be neutralized to prevent, for example, side reactions and/or equipment corrosion during the distillation process.

Neutralisation may be accomplished by treating the acids with a basic material, such as an aqueous alkaline agent. During the neutralization reaction, salts are formed. However, these too can cause problems during the subsequent purification and recovery of phenol and acetone by distillation. For example, salt carryover to the distillation section causes fouling and down time, and thus capacity reduction.

The neutralization of the cleavage product mixture produced in a phenol production process has generally been conducted by adding a caustic agent to the mixture or by adding an aqueous salt solution optionally containing excess caustic agent, as in US 6,066,767. This publication concerns a method for purifying CHP cleavage products in a purification process system. Purification is performed by extracting hydroxy acetone and carbonyl compounds with a water-salt solution at the neutralization stage by adding a caustic agent. US 3,931,339 concerns a process for the removal and neutralization of an acid catalyst and organic acid by-products from the reaction mixture resulting from cumene hydroperoxide cleavage. The process comprises extracting the acids from the organic phase into an aqueous phase and neutralizing them using an aqueous solution of an acidic or neutral inorganic salt, and an excess of alkali metal hydroxide or alkali metal phenate, separating the aqueous phase containing the neutralized acids from the mixture, then contacting the organic phase with an aqueous solution of an acidic or neutral inorganic salt and a weak acid, and thereafter again separating the aqueous phase from the mixture.

GB 743 004 concerns a process for the production of phenol and acetone from CHP, in which the acid and acidic organic impurities in the cleavage mixture are neutralized using an alkaline material. Corresponding salts of the acid and acidic impurities are then partly precipitated in the effluent, whereby they are removed by adding water to the neutralized cleavage mixture in an amount sufficient to cause formation of two phases. The aqueous phase will then contain substantially all of the salts. The salt content of the organic phase will, according to the publication, be less than 0.03% by weight, often as low as about 0.01% by weight.

The conventional processes are generally focused on neutralizing cleavage product mixtures. In some cases, the salts are allowed to be carried over to the next stage of the process, e.g. a distillation. In other cases, the removal of salts is attempted, for example using coalescers. The large amount of salts present in these cleavage product mixtures will, however, cause clogging of coalescers and filters. Further, without addition of excess water before any phase separation step following a neutralization, the neutralized cleavage product mixture will not contain a sufficient amount of water for an aqueous phase to separate from the rest of the mixture.

We have now developed an alternative process for efficiently removing salts from an organic solution which is particularly suitable for use in a phenol production process. In particular, we have developed a process for removing the salts formed during the neutralization of an acid-containing organic solution which involves the use of alternative means for achieving efficient mixing of the neutralised solution with added water and which, subsequently, enables efficient phase separation of the resulting organic and aqueous phases.

Specifically, the present invention concerns a process for the removal of a salt from a salt-containing organic mixture, which process comprises, in a first step, adding water, preferably fresh water, to the salt-containing organic mixture in order to form a water-containing organic mixture, in a second step, allowing the mixture to settle in a settling zone into an organic upper phase and an aqueous lower phase, and in a third step, separating the organic upper phase from the aqueous lower phase. In this process, a water stream is brought into contact with a feed stream which comprises the salt-containing organic mixture during flow of this mixture to the settling zone, for example during its flow in a feed line. Surprisingly, the flow of the resulting mix through the feed line has been found to provide effective mixing of the components and, in turn, efficient separation on settling. In particular, it has been found that an effective removal of salts can be achieved by the use of a feed line which is long enough to provide the desired residence time needed to dissolve particulate matter, e.g. salts, into the water phase.

An apparatus is also described which is adapted for carrying out the process herein described. Specifically, an apparatus is described for neutralizing an acid-containing organic mixture and for removing salt from the resulting neutralized organic mixture.

Considerable advantages are obtained by means of the invention. The present invention provides a simplified process for efficiently removing salts from an organic mixture of components by forming an aqueous phase and an organic phase after thorough mixing of the components, whereby the desired products dissolve in the organic phase and the salts dissolve in the aqueous phase. For example, the process of the invention avoids the need for separate mixing means (e.g. a conventional mixing tank or a mixer) to ensure thorough mixing of the components. In contrast to conventional procedures, the invention relies solely on the use of a conduit (e.g. feed pipe) to provide adequate mixing of the salt-containing organic mixture and water without the need for any additional mixing means. A stream of the salt-containing organic mixture and a water stream enter the conduit (e.g. pipe) separately, i.e. without prior mixing. This conduit feeds directly into the separation zone without passing through any additional mixing means such as a mixing tank or mixer (e.g. a static mixer). By optimizing the conditions and the reagents used in the process, the phase separation can be further optimized. Following separation of the organic and aqueous phases, no significant concentration of salts remains in the organic phase thereby ensuring that no significant concentration of salts reaches any downstream equipment (e.g. distillation apparatus) where clogging could occur.

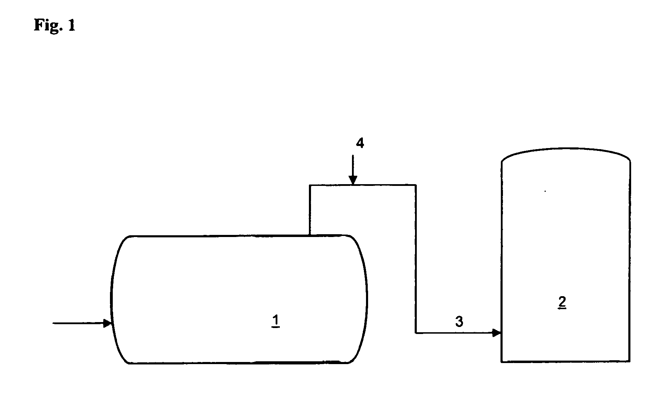

Next, the invention will be described more closely with reference to attached Figure 1 which is a schematic drawing of an apparatus suitable for use in carrying out the process of the present invention. , The present invention concerns a process for the removal of a salt (preferably more than one salt) from a salt-containing organic mixture, preferably from an organic mixture comprising phenol, acetone, eumene and one or more organic or inorganic salts, and optionally one or more further organic or inorganic components. In a first step of the process, water (e.g. fresh water) is added to the salt-containing organic mixture in order to form a water-containing organic mixture. In a second step, the resulting mixture is allowed to settle in a settling zone into an organic upper phase and an aqueous lower phase. Finally, in a third step, the organic upper phase is separated from the aqueous lower phase.

Particularly, the process of the present invention comprises the step of combining separate streams of the salt-containing organic mixture and water in a conduit or feed line and conducting the resulting water-containing mixture to the settling zone of the second step through this conduit (e.g. feed line) in which the components are allowed to mix and the salts are allowed to dissolve into the water. The conduit is typically a feed line or pipe which conveys fluid directly from the neutralisation zone into the separation zone. By the term "directly" it is intended that the feed line should not pass through any additional mixing zone such as a mixing tank or any other mixing means, such as a static mixer.

Suitable conduits include feed pipes conventionally used in industrial processes. In order to ensure adequate mixing of the two solutions within the conduit, this will typically be at least 50 m in length, preferably at least 100 m, more preferably at least 150 m. The upper limit of the length of the conduit is not critical to performance of the invention, but in general it is anticipated that this will not be more than about 500 m. For example, the length of the feed line may be of the order of 100 to 500 m, preferably 150 to 300 m. A pipe having a length of about 200 m is particularly preferred. The cross-sectional diameter of the pipe is of less importance but will typically be in the range of from about 5 cm to about 30 cm, e.g. about 10-20 cm. Within the pipe, fluid flow rates may be adjusted (e.g. using pumps) in order to optimise mixing and/or pressure may be applied to create turbulent mixing. Suitable flow velocities may readily be determined by those of skill in the art, but may be expected to range from 0.5 to 5 ms⁻¹, e.g. 1 to 3 ms⁻¹, preferably about 2 ms⁻¹. Turbulent mixing can generally be achieved at a flow rate of 0.5 ms⁻¹ and above. The residence time within the pipe will be dependent on the size and length of the pipe and the flow velocity, but will generally exceed 30 seconds, preferably 60 seconds, more preferably 90 seconds.

Following mixing of the solutions within the feed pipe, the mixed solution is passed into a settling zone, e.g. a settling tank. Residence times within the settling zone may vary but typically will be at least 12 hours, more preferably at least 24 hours, e.g. up to 48 hours. Once separated, the aqueous lower phase is removed in the third step.

According to a preferred embodiment of the present invention, the salt-containing organic mixture, also referred to herein as "the neutralized organic mixture", is conducted to the first step of the process from a step of neutralizing an organic mixture using an acid or a base, preferably an acid. This mixture to be neutralized may be acidic or basic, but preferably this will be acidic. A particularly suitable acid-containing organic mixture for use in the process herein described is the cleavage product mixture produced during the final cleavage step of the cumene process used in the production of phenol. Such a mixture preferably contains phenol, acetone, cumene and one or more further organic or inorganic components. The mixture has preferably been obtained from a step of acid-catalyzed cleavage of cumene hydroperoxide (CHP).

The process of the present invention is based on a liquid-liquid extraction procedure for separating the components, such as the compounds and the ions, of the salt-containing mixture based on their solution preferences for two different immiscible liquids, preferably water and an organic phase. Based on these preferences, the organic upper phase formed during the second step of the process of the present invention contains substantially all (for example 80 to 95 wt.%, more preferably 90 to 95 wt.%) of the phenol and acetone, whereas the aqueous lower phase contains substantially all of the added water and the salts dissolved therein. The aqueous phase may be expected to contain 80 to 99 wt.% of the added water, preferably 90 to 99 wt.%.

The amount of salt typically present in the salt-containing organic mixture prior to treatment is about 120 to 250 ppm. Following the second step of the process herein described (i.e. settling), the salt content of the organic phase will generally be reduced to 60 to 130 ppm. As a result of carrying out the process of the invention, the salt content of the organic mixture is generally reduced by at least 40%, preferably at least 45%, more preferably by at least 50%. Such a reduction is considered highly significant.

The apparatus suitable for use in the process of the present invention for neutralizing an acid-containing organic mixture and for the subsequent removal of salt from the neutralized organic mixture preferably comprises the following parts (Fig. 1):

| | |
|---|---|
| 1 | neutralization zone |
| 2 | separation zone |
| 3 | line |
| the line further comprising: | |
| 4 | water inlet |

With specific reference to Figure 1, the acid-containing organic mixture may be conducted to at least one neutralization zone 1 for neutralizing the acidic components of the acid-containing organic mixture, and for producing a neutralized salt-containing organic mixture. Subsequently, the neutralized organic mixture may be conducted further to at least one separation zone 2 positioned downstream from the neutralization zone 1, for separating the neutralized organic mixture into an organic phase and an aqueous phase. Between the neutralization zone 1 and the separation zone 2 is positioned a line 3 which is in fluid. connection (preferably direct fluid connection) with the at least one neutralization zone 1 and with the at least one separation zone 2. Thus, the neutralized mixture may be conducted directly from the neutralization zone 1 to the separation zone 2 through the line 3. The line further comprises a water inlet 4 for conducting fresh water into the line 3, which water may be mixed with the neutralized organic mixture before being conducted to the separation zone 2. The exact positioning of water inlet 4 along line 3 can readily be determined by those skilled in the art such that the length of the line 3 which extends from the point of the water inlet 4 to the point of entry into the separation zone is sufficient to ensure adequate mixing of the fluids. In general, this length (i.e. the distance between the water inlet point 4 and the separation zone 2) will be about 100 to 500 m, preferably 150 to 300 m, e.g. about 200 m. Typically, the water inlet 4 will be positioned directly adjacent to the neutralisation zone 1.

Although the invention is primarily described herein in the context of the phenol production process, the process of the present invention may be used to remove salts from any organic mixture of components. Preferably, however, the organic mixture of components is a mixture containing the cleavage products (and by-products) obtained from the cleavage of cumene hydroperoxide. More preferably, the apparatus herein described is arranged downstream from the cumene hydroperoxide cleavage section of a phenol production process. The phenol production process typically comprises process steps wherein phenol and acetone are produced through the oxidation of cumene to cumene hydroperoxide (CHP) and the subsequent steps of concentrating the CHP, cleaving the CHP into phenol, acetone and other cleavage products, neutralizing, washing and desalting the cleavage products and, finally, separating the acetone from the phenol and purifying both desired products.

Preferably, as mentioned above, the cleavage of cumene hydroperoxide into cleavage products (the main ones being phenol and acetone) occurs through an acid-catalyzed reaction making the cleavage product mixture acidic. The acid present in this acid-containing organic mixture is preferably an inorganic acid, and more preferably sulfuric acid. The concentration of added acid in the cleavage product mixture is about 0.005-0.2 wt.%, preferably 0.01-0.15 wt.%.

According to a preferred embodiment of the present invention, the added acids are neutralized by adjusting the pH of the mixture to about 4.5 - 6.5 using an aqueous alkaline solution. This neutralization is carried out in a neutralization zone 1 using an aqueous alkaline solution, which preferably is a solution of sodium hydroxide (NaOH). The sodium hydroxide may for example be conducted to the neutralizing circulation as an aqueous solution with a concentration of 15-35%, preferably 20-25%. The water concentration in the neutralized mixture is generally about 8-12 wt.% (hydrocarbons are saturated with water).

From the neutralization zone 1, the neutralized mixture is conducted through a line 3 to the separation zone 2. In order to increase the amount of dissolved salts and to ease the formation of two phases, 1-5 wt.%, preferably 1-2 wt.% water (preferably salt-free water) is added into the line 3. The water may be either fresh water or circulated (i.e. recycled) water. In the line 3, the mixture and the water are mixed thoroughly, whereafter they are conducted to the separation zone 2 for settling. Finally, the two phases, the organic phase and the aqueous phase, are separated.

The water used in the process of the invention is preferably fresh water, but may be recycled water. Preferably, the water used in the process is water essentially free from acidic and alkaline components and/or free from salts. Preferred for use in the invention is water condensate which contains essentially zero acidic or alkaline compounds and which is essentially salt-free (i.e. these substances are present in an amount which is below normal detection limits). Where any acidic or alkaline components are present these will generally be present in an amount of less than 10 ppm, preferably less than 0.1 ppm. Where any salts are present these should be present in an amount of less than 1 ppm, e.g. less than 0.1 ppm.

The concentration of salt in the neutralized salt-containing organic mixture will generally be about 50-300 ppm, whereas the concentration of salt in the organic phase separated from the salt-containing organic mixture in the third step of the process will typically be about 30-150 ppm.

According to a preferred embodiment of the present invention, a coalescer is placed downstream from the settling zone. In the coalescer, the concentration of salt in the product mixture may be further decreased to about 5ppm or even below.

The temperature during the neutralization and the salt-removal of the present invention will generally be maintained at 30-50°C, preferably at about 40°C.

The following non-limiting Example is intended to further illustrate the invention:

### Example

Cleavage products from a phenol production process containing 48-50 wt.% phenol, 35 wt.% acetone, 11-12 wt.% cumene, 1-1.5 wt.% water and 0.15 wt.% sulfuric acid are neutralised by the addition of a solution of sodium hydroxide (25%). Sodium hydroxide is added in an amount sufficient to adjust the pH to 6.5. The temperature is kept at 40°C and residence time in the neutralisation zone is about 1.5 hours.

The neutralised salt-containing mixture is conducted through a 200 m feed line (diameter: 10 cm) at a flow velocity of 2 ms⁻¹ to which 1 wt.% fresh water (water condensate) is added via a water inlet positioned adjacent to the neutralisation zone. After thorough mixing in the feed line (residence time: about 100 seconds) the solution is passed into a separation zone or settling tank where the two phases are allowed to separate for about 24 hours at a temperature of about 40°C. The two phases are then separated and the salt concentration in the organic phase is about 50 ppm.

## Claims

1. A process for the removal of a salt from a salt-containing organic mixture, which process comprises
- in a first step, adding water to the salt-containing organic mixture in order to form a water-containing organic mixture,
- in a second step, allowing the water-containing organic mixture to settle in a settling zone into an organic upper phase and an aqueous lower phase,
- in a third step, removing the aqueous phase from the organic phase,
wherein
- the water-containing organic mixture in the first step is conducted through a line, in which the components of the mixture are allowed to mix and one or more salts contained therein are allowed to dissolve into the water, directly into the separation zone without passing through any additional mixing means.

2. The process of claim 1, wherein the flow rate within the pipe is at least 0.5 ms⁻¹ and the residence time of the water-containing organic mixture in the pipe is at least 30 seconds.

3. The process of claim 1 or claim 2, wherein the salt-containing organic mixture contains phenol, acetone, cumene and one or more organic or inorganic salts and one or more further organic or inorganic components.

4. The process of any one of claims 1 to 3, wherein the salt-containing mixture is conducted to the first step from a step of neutralizing an acid-containing organic mixture.

5. The process of claim 4, wherein the organic mixture contains phenol, acetone, cumene and one or more further organic or inorganic components.

6. The process of claim 4 or claim 5, wherein the step of neutralization of the acid-containing organic mixture is carried out using an aqueous alkaline solution.

7. The process of claim 6, wherein the step of neutralization of the acid-containing organic mixture is carried out by adding sodium hydroxide (NaOH) to the acid-containing organic mixture.

8. The process of any one of claims 4 to 7, wherein the acid-containing organic mixture is obtained from a step of acid-catalysed cleavage of cumene hydroperoxide.

9. The process of any one of claims 3 to 8, wherein the water-containing organic mixture Is allowed to settle In a settling zone into an organic upper phase containing substantially all of the phenol and acetone, and an aqueous lower phase containing substantially all of the saft.

10. The process of any preceding claim, wherein the water is fresh water or recycled water.

11. The process of claim 10, wherein the water is water which is essentially free from acidic and alkaline components, or water free from salts.

12. The process of any preceding claim, wherein the concentration of salt in the salt-containing mixture is decreased from a value of 50-300ppm in the salt-containing mixture to a value of 30-150 ppm in the organic upper phase formed in the second step.

13. The process of any preceding claim, wherein the concentration of salt in the salt-containing mixture is further decreased to a value of 5ppm or below by passing the mixture through a coalescer.

14. The process of any preceding claim, wherein the temperature Is maintained at 30-50°C, preferably at 40°C.

15. Use of the process of any one of claims 1-14 for removing salt from a neutralized organic mixture obtained by cleaving cumene hydroperoxide to produce a cleavage product mixture and subsequently neutralizing the cleavage product mixture.

## Patentansprüche

1. Verfahren zur Entfernung eines Salzes aus einer salzhaltigen organischen Mischung, wobei das Verfahren umfasst:
- in einem ersten Schritt das Zugeben von Wasser zu der salzhaltigen organischen Mischung, um eine wasserhaltige organische Mischung zu bilden,
- in einem zweiten Schritt das Absetzenlassen der wasserhaltigen organischen Mischung in einer Absetzzone in eine organische obere Phase und eine wässrige untere Phase,
- in einem dritten Schritt das Entfernen der wässrige Phase von der organischen Phase,
wobei
- die wasserhaltige organische Mischung in dem ersten Schritt durch eine Leitung, in der es den Bestandteilen der Mischung erlaubt wird, sich zu mischen, und es einem oder mehreren darin enthaltenen Salzen erlaubt wird, sich im Wasser zu lösen, direkt in die Trennzone geleitet wird, ohne durch zusätzliche Mischmittel geleitet zu werden.

2. Verfahren gemäß Anspruch 1, wobei die Durchflussrate in dem Rohr mindestens 0,5 ms⁻¹ beträgt und die Verweilzeit der wasserhaltigen organischen Mischung in dem Rohr mindestens 30 Sekunden beträgt.

3. Verfahren gemäß Anspruch 1 oder Anspruch 2, wobei die salzhaltige organische Mischung Phenol, Aceton, Cumol und ein oder mehrere organische oder anorganische Salze und einen oder mehrere organische oder anorganische Bestandteile enthält.

4. Verfahren gemäß einem der Ansprüche 1 bis 3, wobei die salzhaltige Mischung von einem Schritt des Neutralisierens einer säurehaltigen organischen Mischung zu dem ersten Schritt hingeführt wird.

5. Verfahren gemäß Anspruch 4, wobei die organische Mischung Phenol, Aceton, Cumol und einen oder mehrere organische oder anorganische Bestandteile enthält.

6. Verfahren gemäß Anspruch 4 oder Anspruch 5, wobei der Schritt des Neutralisierens der säurehaltigen organischen Mischung unter Verwendung einer wässrigen alkalischen Lösung durchgeführt wird.

7. Verfahren gemäß Anspruch 6, wobei der Schritt des Neutralisierens der säurehaltigen organischen Mischung durch Zugabe von Natriumhydroxid (NaOH) zu der säurehaltigen organischen Mischung durchgeführt wird.

8. Verfahren gemäß einem der Ansprüche 4 bis 7, wobei die säurehaltige organische Mischung aus einem Schritt der säurekatalysierten Spaltung von Cumolhydroperoxid erhalten wird.

9. Verfahren gemäß einem der Ansprüche 3 bis 8, wobei es der wasserhaltigen organischen Mischung in einer Absetzzone erlaubt wird, sich in eine organische obere Phase, die im Wesentlichen das gesamte Phenol und Aceton enthält, und in eine wässrige untere Phase, die im Wesentlichen das gesamte Salz enthält, abzusetzen.

10. Verfahren gemäß einem der vorhergehenden Ansprüche, wobei es sich bei dem Wasser um frisches Wasser oder um recyceltes Wasser handelt.

11. Verfahren gemäß Anspruch 10, wobei es sich bei dem Wasser um Wasser, das im Wesentlichen frei von sauren und alkalischen Bestandteilen ist, oder um Wasser, das frei von Salzen ist, handelt.

12. Verfahren gemäß einem der vorhergehenden Ansprüche, wobei die Salzkonzentration in der salzhaltigen Mischung von einem Wert von 50-300 ppm in der salzhaltigen Mischung bis auf einen Wert von 30-150 ppm in der organischen oberen Phase, die in dem zweiten Schritt gebildet wird, verringert wird.

13. Verfahren gemäß einem der vorhergehenden Ansprüche, wobei die Salzkonzentration in der salzhaltigen Mischung weiter auf einen Wert von 5 ppm oder darunter verringert wird, indem die Mischung durch einen Tropfenabscheider geleitet wird.

14. Verfahren gemäß einem der vorhergehenden Ansprüche, wobei die Temperatur bei 30-50 °C, vorzugsweise bei 40 °C, gehalten wird.

15. Verwendung des Verfahrens gemäß einem der Ansprüche 1-14 zur Entfernung von Salz aus einer neutralisierten organischen Mischung, die durch Spalten von Cumolhydroperoxid zur Herstellung einer Spaltproduktmischung und anschließendes Neutralisieren der Spaltproduktmischung erhalten wird.

## Revendications

1. Procédé d'élimination d'un sel d'un mélange organique contenant un sel, ledit procédé comprenant les étapes consistant à :
- dans une première étape, ajouter de l'eau au mélange organique contenant un sel pour former un mélange organique contenant de l'eau,
- dans une deuxième étape, permettre au mélange organique contenant de l'eau de se déposer dans une zone de dépôt en une phase supérieure organique et une phase inférieure aqueuse, et
- dans une troisième étape, séparer la phase aqueuse de la phase organique,
dans lequel :
- le mélange organique contenant de l'eau de la première étape est acheminé à travers une conduite dans laquelle on laisse les composants du mélange se mélanger et un ou plusieurs des sels qui y sont contenus se dissoudre dans l'eau, directement dans la zone de séparation sans passer par des moyens de mélange supplémentaires quelconques.

2. Procédé selon la revendication 1, dans lequel le débit à l'intérieur de la conduite est d'au moins 0, 5 ms⁻¹ et le temps de séjour du mélange organique contenant de l'eau dans la conduite est d'au moins 30 secondes.

3. Procédé selon la revendication 1 ou la revendication 2, dans lequel le mélange organique contenant un sel contient du phénol, de l'acétone, du cumène et un ou plusieurs sels organiques ou inorganiques et un ou plusieurs autres composants organiques ou inorganiques.

4. Procédé selon l'une quelconque des revendications 1 à 3, dans lequel le mélange contenant un sel est acheminé à la première étape depuis une étape de neutralisation d'un mélange organique contenant un acide.

5. Procédé selon la revendication 4, dans lequel le mélange organique contient du phénol, de l'acétone, du cumène et un ou plusieurs autres composants organiques ou inorganiques.

6. Procédé selon la revendication 4 ou la revendication 5, dans lequel l'étape de neutralisation du mélange organique contenant un l'acide est effectuée en utilisant une solution alcaline aqueuse.

7. Procédé selon la revendication 6, dans lequel l'étape de neutralisation du mélange organique contenant un acide est effectuée en ajoutant de l'hydroxyde de sodium (NaOH) au mélange organique contenant un acide.

8. Procédé selon l'une quelconque des revendications 4 à 7, dans lequel le mélange organique contenant un acide est obtenu à partir d'une étape de clivage d'hydroperoxyde de cumène catalysé par un acide.

9. Procédé selon l'une quelconque des revendications 3 à 8, dans lequel on laisse le mélange organique contenant de l'eau se déposer dans une zone de dépôt en une phase supérieure organique contenant sensiblement la totalité du phénol et de l'acétone et une phase inférieure aqueuse contenant sensiblement la totalité du sel.

10. Procédé selon l'une quelconque des revendications précédentes, dans lequel l' eau est formée d'eau fraîche ou d'eau recyclée.

11. Procédé selon la revendication 10, dans lequel l'eau est de l'eau qui est sensiblement exempte de composants acides et alcalins ou de l'eau exempte de sels.

12. Procédé selon l'une quelconque des revendications précédentes, dans lequel la concentration de sel dans le mélange contenant un sel est réduite d'une valeur de 50 à 300 ppm dans le mélange contenant un sel à une valeur de 30 à 150 mm dans la phase supérieure organique formée dans la seconde étape.

13. Procédé selon l'une quelconque des revendications précédentes, dans lequel la concentration de sel dans le mélange contenant un sel est encore réduite à une valeur de 5 ppm ou en dessous en faisant passer le mélange à travers un appareil de coalescence.

14. Procédé selon l'une quelconque des revendications précédentes, dans lequel la température est maintenue à un niveau de 30 à 50 °C, de préférence à 40 °C.

15. Utilisation du procédé selon l'une quelconque des revendications 1 à 14 pour éliminer un sel d'un mélange organique neutralisé obtenu par clivage d'hydroperoxyde de cumène afin d'obtenir un mélange de produits de clivage et en neutralisant ensuite le mélange de produits de clivage.
